# EUROPEAN PATENT SPECIFICATION

(11) **EP 3 402 562 B1**
(45) Date of publication and mention of the grant of the patent: **21.12.2022**
(21) Application number: 17703522.7
(22) Date of filing: 13.01.2017
(51) Int. Cl.: A61M 25/01

(54) **SLOTTED TUBE WITH PLANAR STEERING**
GESCHLITZTES ROHR MIT PLANARER LENKUNG
TUBE FENDU À GUIDAGE PLAN

(30) Priority: 15.01.2016 US 201662279381 P
(43) Date of publication of application: 21.11.2018
(73) Proprietor: Boston Scientific Scimed, Inc., Maple Grove, MN 55311 (US)
(72) Inventor: GRONEBERG, David D., Plymouth Minnesota 55441 (US); HANSON, Brian J., Shoreview Minnesota 55126 (US); EGGERT, Joel T., Plymouth Minnesota 55442 (US); MARECKI, Andrew T., Shrewsbury Massachusetts 01545 (US); FETTIG, Jonathan P., Blaine Minnesota 55449 (US)
(74) Representative: Vossius & Partner Patentanwälte Rechtsanwälte mbB
(86) International application number: PCT/US2017/013434
(87) International publication number: WO 2017/123945

(56) References cited:
- EP-A1- 2 740 400
- US-A- 5 228 441
- US-A1- 2005 288 656
- US-A1- 2009 254 000
- US-A1- 2010 010 437
- US-A1- 2013 096 553
- US-A1- 2014 053 940

## Description

### Cross-Reference To Related Applications

This application claims the benefit of priority under 35 U.S.C. §119 to U.S. Provisional Application No. 62/279,381, filed January 15, 2016.

### Technical Field

The present disclosure pertains to medical devices, and methods for manufacturing medical devices. More particularly, the present disclosure pertains to elongated intracorporeal medical devices including a tubular member connected with other structures, and methods for manufacturing and using such devices.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

### Summary

The invention is defined by independent claim 1 and the subsequent dependent claims 2 - 9.

The disclosure is directed to several alternative designs, materials and methods of manufacturing medical device structures and assemblies, and uses thereof. An elongate medical device is disclosed. The elongate medical device comprises:
an elongate tubular body extending from a proximal region to a distal region and defining a lumen extending there between, the elongate tubular body including a proximal portion configured for isotropic bending and a distal portion configured for anisotropic bending;
a first plurality of steering wire guides formed along a first side of the distal region of the elongate tubular body;
a first steering wire extending within the lumen and having a distal end secured to the distal region of the elongate tubular body, the first steering wire passing through the first plurality of steering wire guides such that the first steering wire is held along the first side of the distal region;
a second plurality of steering wire guides formed along a second side of the distal region of the elongate tubular body; and
a second steering wire extending within the lumen and having a distal end secured to the distal region of the elongate tubular body, the second steering wire passing through the second plurality of steering wire guides such that the second steering wire is held along the second side of the distal region.

The proximal portion configured for isotropic bending comprises a plurality of slots cut into the elongate tubular body, the plurality of slots including a first pair of opposed slots at a first position on the elongate tubular body and a second pair of opposed slots adjacent to the first pair of slots and arranged at a second position axially offset and rotated from the first position.

The distal portion configured for anisotropic bending comprises a plurality of slots cut into the elongate tubular body, the plurality of slots including a first slot cut into the first side of the elongate tubular body and a second slot cut into the elongate tubular body from the second side of the elongate tubular body, the second slot axially offset from the first slot.

Additionally to the embodiment above, the first slot and the second slot each have a cutting depth that exceeds half of a diameter of the elongate tubular body such that the first slot overlaps the second slot, thereby forming a first bending hinge line extending along the elongate tubular body and a second bending hinge line extending along the elongate tubular body.

Additionally to any of the embodiments above, when the elongate tubular body is in a linear configuration, a first plane extends through the first side of the elongate tubular body and the second side of the elongate tubular body, and a second plane orthogonal to the first plane extends through the first bending hinge line and the second bending hinge line.

Additionally to any of the embodiments above, the distal portion of the elongate tubular body preferentially bends within the first plane and is resistant to bending within the second plane.

Additionally to any of the embodiments above, at least some of the first plurality of steering wire guides comprise a portion of the elongate tubular body, between adjacent slots, that is bent to form a loop extending into the lumen.

Additionally to any of the embodiments above, at least some of the second plurality of steering wire guides comprise a portion of the elongate tubular body, between adjacent slots, that is bent to form a loop extending into the lumen.

Additionally to any of the embodiments above, the elongate tubular body comprises an intermediate portion disposed between the proximal portion and the distal portion.

Additionally to any of the embodiments above, the elongate tubular body includes a single tube forming the proximal portion and the distal portion of the elongate tubular body.

A steerable medical device is disclosed. The steerable medical device comprises:
a tubular member extending from a proximal region to a distal region and defining a lumen extending there between;
one or more steering wire guides formed into a first side of the distal region of the tubular member;
one or more steering wire guides formed into a second side of the distal region of the tubular member;
a first steering wire extending within the lumen and having a distal end secured to the distal region of the tubular member, the first steering wire passing through the one or more steering wire guides on the first side of the distal region; and
a second steering wire extending within the lumen and having a distal end secured to the distal region of the tubular member, the second steering wire passing the one or more steering wire guides on the second side of the distal region.

Alternatively or additionally to any of the embodiments above, the distal region of the tubular member comprises a plurality of slots that are cut into the tubular member.

Additionally to any of the embodiments above, at least one of the one or more steering wire guides formed into the first side of the distal region of the tubular member are formed by bending a portion of the tubular member, between adjacent slots, into a loop extending into the lumen.

Additionally to any of the embodiments above, at least one of the one or more steering wire guides formed into the second side of the distal region of the tubular member are formed by bending a portion of the tubular member, between adjacent slots, into a loop extending into the lumen.

Additionally to any of the embodiments above, the plurality of slots are formed in a pattern that provides the distal region of the tubular member with anisotropic bending.

Additionally to any of the embodiments above, the proximal portion of the tubular member comprises a plurality of slots that are cut into the tubular member and are configured to provide the proximal portion of the tubular member with isotropic bending.

An exemplary elongate medical device is disclosed. The elongate medical device comprises:
an elongate tubular body extending from a proximal region to a distal region and defining a lumen extending there between, the elongate tubular body including a proximal portion configured for anisotropic bending and a distal portion configured for isotropic bending;
the proximal portion comprising a plurality of slots cut into the elongate tubular body, the plurality of slots including a first pair of opposed slots at a first position on the elongate tubular body and a second pair of opposed slots adjacent to the first pair of opposed slots and arranged at a second position axially offset and rotated from the first position;
the distal portion comprising a plurality of slots cut into the elongate tubular body, the plurality of slots including a first slot cut into a first side of the elongate tubular body and a second slot cut into the elongate tubular body from a second side of the elongate tubular body, the second slot axially offset from the first slot; and
the first slot and the second slot formed in the distal portion each having a cutting depth that exceeds half of a diameter of the elongate tubular body such that the first slot overlaps the second slot, thereby forming a first bending hinge line extending along the elongate tubular body and a second bending hinge line extending along the elongate tubular body;
wherein when the elongate tubular body is in a linear configuration, a first plane extends through the first side of the elongate tubular body and the second side of the elongate tubular body, and a second plane orthogonal to the first plane extends through the first bending hinge line and the second bending hinge line.

Additionally to the example above, the distal portion of the elongate tubular body preferentially bends within the first plane and is resistant to bending within the second plane.

Additionally to any of the examples above, the elongate medical device comprises:
a first plurality of steering wire guides formed along a first side of the distal region of the elongate tubular body; and
a first steering wire extending within the lumen and having a distal end secured to the distal region of the elongate tubular body, the first steering wire passing through the first plurality of steering wire guides such that the first steering wire is held along the first side of the distal region.

Additionally of additionally to any of the examples above, the elongate medical device comprises:
a second plurality of steering wire guides formed along a second, opposing, side of the distal region of the elongate tubular body; and
a second steering wire extending within the lumen and having a distal end secured to the distal region of the elongate tubular body, the second steering wire passing through the second plurality of steering wire guides such that the second steering wire is held along the second side of the distal region.

The above summary is not intended to describe each embodiment or every implementation of the present disclosure. Advantages and attainments, together with a more complete understanding of the disclosure, will become apparent and appreciated by referring to the following description and claims taken in conjunction with the accompanying drawings.

### Brief Description of the Figures

The disclosure may be more completely understood in consideration of the following description of various illustrative embodiments in connection with the accompanying drawings, in which:
Figure 1 is a schematic illustration of a steerable elongate medical device in accordance with an embodiment of the disclosure;
Figure 2 is a view of the steerable elongate medical device of Figure 1, shown with its distal region curved in a first direction;
Figure 3 is a view of the steerable elongate medical device of Figure 1, shown with its distal region curved in a second direction;
Figure 4 illustrates a portion of the steerable elongate medical device of Figure 1;
Figure 5 is a plan view of a portion of an elongate tubular member forming a portion of the steerable elongate medical device of Figure 1;
Figure 6 is a schematic cross-sectional view of the elongate tubular member of Figure 5;
Figure 7 shows a distal portion of the steerable elongate medical device of Figure 1 in a linear configuration; and
Figure 8 provides a view of a distal portion of the steerable elongate medical device of Figure 1, showing the steering wires.

While the disclosure is amenable to various modifications and alternative forms, specifics thereof have been shown by way of example in the drawings and will be described in detail. It should be understood, however, that the intention is not to limit aspects of the disclosure to the particular illustrative embodiments described. On the contrary, the intention is to cover all modifications falling within the scope of the appended claims.

### Description

The following description should be read with reference to the drawings in which similar elements in different drawings are numbered the same. The description and the drawings, which are not necessarily to scale, depict illustrative embodiments and are not intended to limit the scope of the disclosure.

All numbers are herein assumed to be modified by the term "about", unless the content clearly dictates otherwise. The recitation of numerical ranges by endpoints includes all numbers subsumed within that range (e.g., 1 to 5 includes 1, 1.5, 2, 2.75, 3, 3.80, 4, and 5).

As used in this specification and the appended claims, the singular forms "a", "an", and "the" include the plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is contemplated that the feature, structure, or characteristic may be applied to other embodiments whether or not explicitly described unless clearly stated to the contrary.

The disclosure pertains generally to elongate medical devices and more particularly to elongate medical devices that are configured to be steerable, e.g., at least a distal portion of the elongate medical device may be curved in a desired direction in response to a steering input provided within a proximal end or portion of the elongate medical device. It will be appreciated that reference to a steerable elongate medical device, such as a steerable catheter, is distinct from an elongate medical device such as a catheter having a predetermined or pre-formed distal curvature. A steerable medical device as referenced herein refers to an elongate medical device, such as a catheter, that can be caused to curve or otherwise change from a linear configuration. In some embodiments, a steerable medical device may refer to an elongate medical device having a predetermined or preformed curved configuration that is configured to enable a user to further alter the distal curvature via one or more proximally-applied steering inputs.

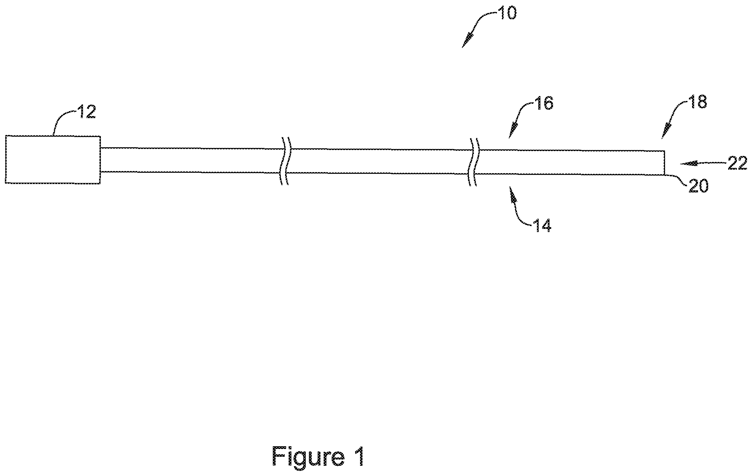

Figure 1 is a schematic view of a steerable catheter 10. While the disclosure generally refers to the steerable catheter 10, it will be appreciated that the steerable catheter 10, or components thereof, may be applicable to a large number of types and applications of catheters and other elongate medical devices. For example, the steerable catheter 10 may represent a catheter having cardio applications, biliary applications, urology applications, and electrophysiology (EP) applications, to provide several illustrative but non-limiting examples. The steerable catheter 10 includes a handle or hub 12 and an elongate shaft 14 that extends distally from the hub 12. The elongate shaft 14 includes a proximal region 16 and a distal region 18. It will be appreciated that proximal and distal are relative terms, simply meaning that the distal region 18 is distal of the proximal region 16, and/or that the proximal region 16 is proximal of the distal region 18. The proximal region 16 and/or the distal region 18 may have any particular length, for example. In some embodiments, for example, the proximal region 16 may extend back to a position at or near the hub 12.

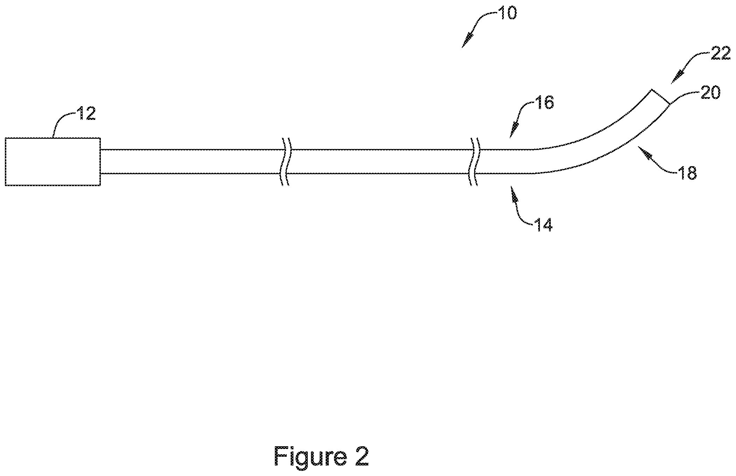

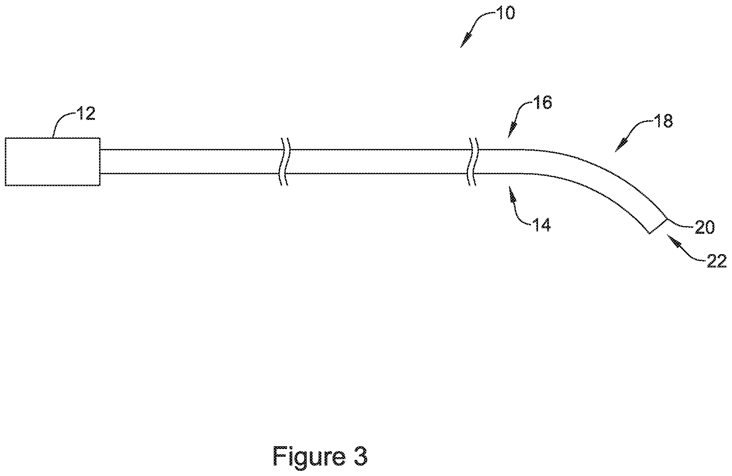

The steerable catheter 10 is steered, or exhibits a curvature within the distal region 18, in response to a steering input provided at or near the hub 12. steering wires (not illustrated in Figures 1-3) extend within the elongate shaft 14 from a position at or near the hub 12 to a position at or near a distal end 20 of the elongate shaft 14. the elongate shaft 14 defines a lumen 22 extending through the elongate shaft 14, and the steering wires extend through the lumen 22. There are at least two steering wires. Pulling on a first steering wire, relative to the second steering wire, may cause the steerable catheter 10 to bend in a first direction, such as that illustrated for example in Figure 2. Conversely, pulling on the second steering wire may cause the steerable catheter 10 to bend in a second direction, such as that illustrated for example in Figure 3.

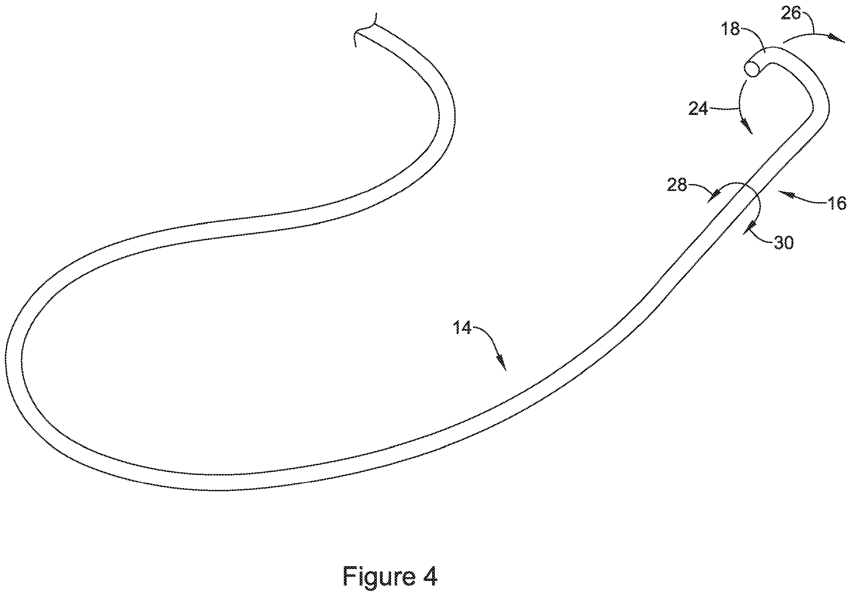

Figure 4 provides a view of a portion of the elongate shaft 14. the proximal region 16 is configured to provide isotropic bending, meaning that the proximal region 16 is equally or substantially equally flexible in any direction. In some examples, having an anisotropic proximal region 16 may be beneficial in navigating tortuous passages within the vasculature, for example. the distal region 18 is configured to provide anisotropic bending, meaning that the distal region 18 may be predisposed to bend more easily in some directions and less easily in other directions. In some examples, the distal region 18 may be predisposed to exhibit planar bending, e.g., the distal region 18 will easily bend in a first plane but resist bending in another plane that may, for example, be orthogonal to the first plane. In Figure 4, arrows 24 and 26 illustrate the first plane in which the distal region 18 is configured to bend. For example, as illustrated in Figure 4, the distal region 18 is curved in the direction indication by the arrow 24.

While the elongate shaft 14 is highly flexible, and thus may be advanced through difficult anatomy such as the vasculature, it will be appreciated that the elongate shaft 14 remains torqueable. A pair of arrows 28 and 30 illustrate how the distal region 18, whether in a curved configuration or in a linear configuration, may be rotated by applying a torque to the elongate shaft 14. In some cases, the combination of curving the distal region 18 in either a direction indicated by the arrow 24 or the arrow 26, in combination with rotating the distal region 18, may provide a desired level of control and precision when advancing the steerable catheter 10 to a desired anatomical location. the elongate shaft 14 is or otherwise includes a elongate tubular member including one or more slot patterns, as described herein, to provide steerability and other desired characteristics.

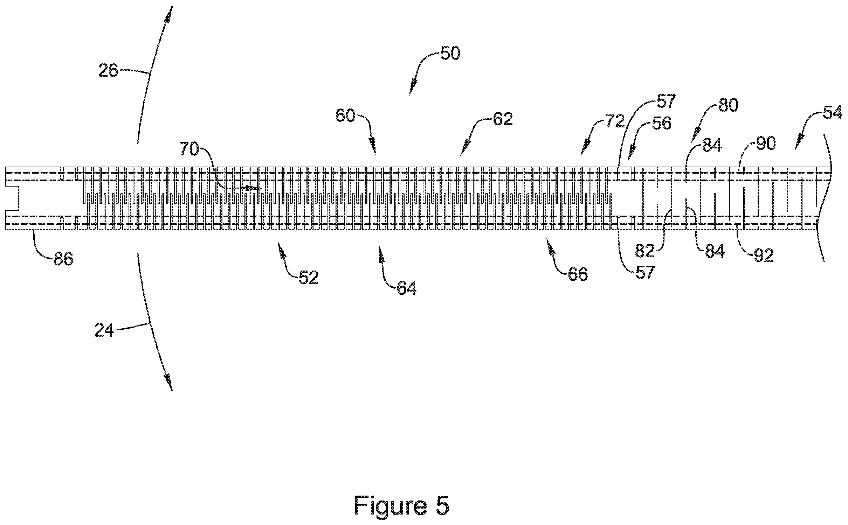

Figure 5 is a plan view of a portion of an elongate tubular member 50. the elongate tubular member 50 forms at least a portion of the elongate shaft 14 (Figure 4). The elongate tubular member 50 is considered as including a distal region 52 and a proximal region 54. In an example, the distal region 52 of the elongate tubular member 50 corresponds to the distal region 18 of the steerable catheter 10. In an example, the proximal region 54 corresponds to the proximal region 16 of the steerable catheter 10. In some embodiments, the elongate tubular member 50 may include an intermediate portion 56 that is disposed between the distal region 52 and the proximal region 54. In some cases, the intermediate portion 56 provides a transition between the flexibility characteristics of the distal region 52 and the flexibility characteristics of the proximal region 54. In some embodiments, the intermediate portion 56 provides a transition in a cutting pattern, as discussed herein, between the distal region 52 and the proximal region 54, particularly in embodiments when the distal region 52 and the proximal region 54 are parts of a single tube forming the elongate tubular member 50.

The distal region 52 includes a cutting pattern that provides the distal region 52 with anisotropic bending characteristics in which the distal region 52 is configured to bend easily in the direction indicated by the arrows 24 and 26 and to resist bending in other directions, particularly in directions that are orthogonal to the direction indicated by the arrows 24 and 26. As can be seen, the distal region 52 includes a plurality of slots 60 cut into a first side 62 of the elongate tubular member 50 and a plurality of slots 64 that are cut into a second side 66 of the elongate tubular member 50. As can be seen, the first side 62 and the second side 66 are about 180 degrees apart from each other.

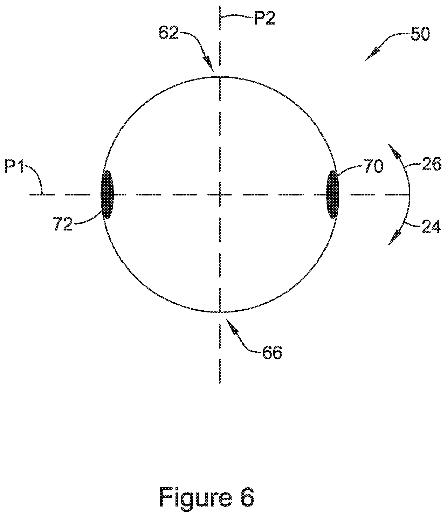

It can be seen that a particular slot of the plurality of slots 60 alternates with a particular slot of the plurality of slots 64. For each slot 60, there is at least one slot 64. In some embodiments, as illustrated, a particular slot 60 has a cutting depth that exceeds half of a diameter of the elongate tubular member 50 from the first side 62 and a particular slot 64 has a cutting depth that exceeds half of the diameter of the elongate tubular member 50 from the second side 64, thereby overlapping. As a result, a first bending hinge line 70 is formed. It will be appreciated that a second bending hinge line 72, not visible in this view, is also formed. as shown in Figure 6. Figure 6 is a schematic cross-sectional view illustrating a first plane P1 extending through the first bending hinge line 70 and the second bending hinge line 72. An orthogonal plane P2 extends through the first side 62 of the elongate tubular member 50 and the second side 66 of the elongate tubular member 60. It will be appreciated that the elongate tubular member 50, if pictured coming out of the page, can easily bend up and down relative to the plane PI, as indicated by the arrows 24 and 26. The elongate tubular member 50 is less likely to bend, if pictured coming out of the page, left and right relative to the plane P2.

Returning to Figure 5, the proximal region 54 is considered as having a slot cutting pattern that provides the proximal region 54 with isotropic bending characteristics. This means that the proximal region 54 may, for example, bend just as easily in any direction. A plurality of slots 80 are cut into the elongate tubular member 50 within the proximal region 54. the plurality of slots 80 include for example a first pair of slots 82 and a second pair of slots 84. the second pair of slots 84 are axially offset and rotated relative to the first pair of slots 82. In this view, one of the first pair of slots 82 is visible while the second of the first pair of slots 82 is on the opposing side and is not seen in this Figure. Because the second pair of slots 84 are rotated relative to the first pair of slots 82, both of the second pair of slots 84 are visible. In some cases, the intermediate region 56 has a cutting pattern selected to provide an appropriate transition in flexibility and/or other desired characteristics between the distal region 52 and the proximal region 54. In an example, the intermediate region 56 may include one or more slots 57 that may be wider and shallower than the slots in the proximal region 54 and/or the distal region 52.

In some embodiments, as illustrated, the elongate tubular member 50 may have a distal end 86. In some cases, the cutting pattern shown within the distal region 52 may extend all the way to the distal end 86. In some instances, the cutting pattern may stop short of the distal end 86. The distal end 86 may, for example, be configured to permit attachment of a distal tip (not illustrated), such as for example an atraumatic tip.

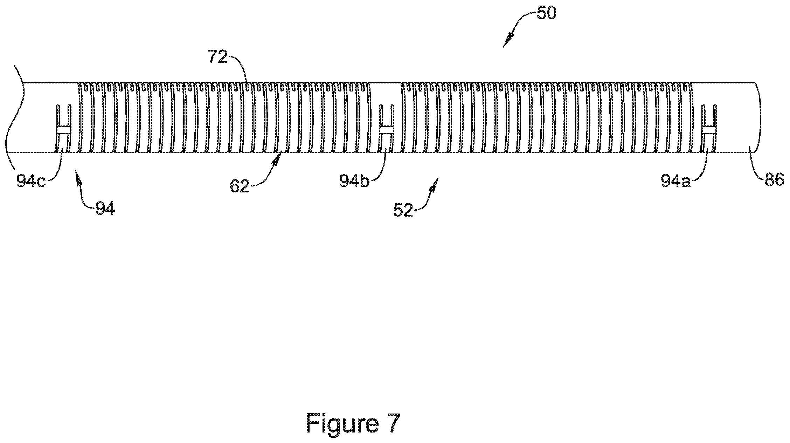

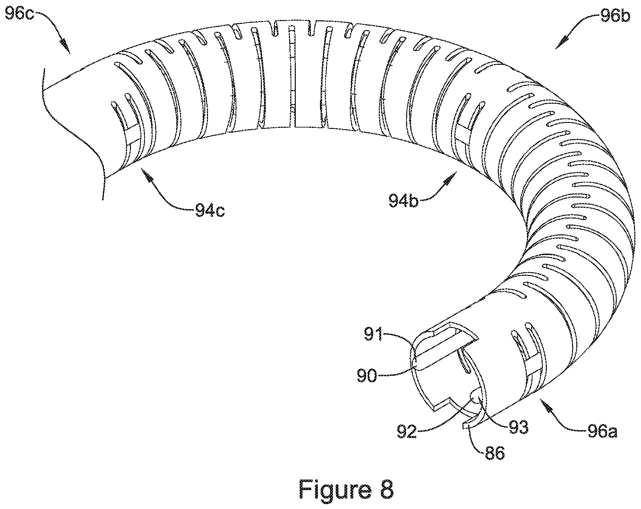

Figures 7 and 8 illustrate features of the steerable catheter 10 that enable steering. Figure 7 provides a view of the distal region 52 of the elongate tubular member 50 in which the elongate tubular member 50 has been rotated relative to its orientation in Figure 5 while in a linear configuration. Figure 8 provides a curved view of the distal region 52 of the elongate tubular member 50 and illustrates the steering wires. In Figure 5, a first steering wire 90 and a second steering wire 92 are shown in phantom. In some cases, it may be desirable to control where the first and second steering wires 90 and 92 are within the lumen 22 (Figure 1). as illustrated in Figures 7 and 8, steering wire guides are provided to limit side to side movement of the first and second steering wires 90 and 92 while permitting free axial movement thereof.

with reference to Figures 7 and 8, a first plurality of steering wire guides 94a, 94b, 94c are formed along the first side 62 of the elongate tubular member 50 and a second plurality of steering wire guides 96a, 96b, 96c are formed along the second side 66, although only the steering wire guide 96a is visible in Figure 8. It will be appreciated that the first steering wire 90 may be slidingly disposed within the first plurality of steering wire guides 94a, 94b, 94c and the second steering wire 92 may be slidingly disposed within the second plurality of wire guides 96a, 96b, 96c. The first steering wire 90 and/or the second steering wire 92 may be secured at their distal ends 91 and 93, respectively, to the distal end 86 of the elongate tubular member 50 and may extend proximally to the hub 12 (Figure 1). In some instances, the distal ends 91, 93 of the steering wires 90, 92 may be welded, soldered, crimped or adhesively secured to the distal end 86.

The first plurality of wire guides 94a, 94b, 94c and/or the second plurality of wire guides 96a, 96b, 96c may be formed in any suitable manner or technique. In some cases, at least some of the wire guides 94a, 94b, 94c and 96a, 96b, 96c may be formed by bending in a portion of the elongate tubular member 50 that is between adjacent slots. As a result, the steering wire guides 94 and 96a, 96b, 96c may extend into the lumen 22 (Figure 1), and thus are able to accommodate the first steering wire 90 and the second steering wire 92 that are within the lumen 22. In some cases, the wire guides 94a, 94b, 94c and 96a, 96b, 96c may be formed from outside the elongate tubular member 50, which in some cases may provide manufacturing advantages.

While the elongate shaft 14 has been illustrated as being formed from the elongate tubular member 50, it will be appreciated that in an example the steerable catheter 10 (Figure 1) may include additional structures and/or layers. For example, at least part of the elongate shaft 14 may include a polymeric covering or coating to render the lumen 22 fluid tight. In an example, if the elongate shaft 14 is made of a non-radiopaque material, the steerable catheter 10 may include one or more radiopaque marker bands. The steerable catheter 10 may include additional structures such as braid, coils and the like.

In some embodiments, various embodiments of arrangements and configurations of slots are also contemplated that may be used in addition to what is described above . For simplicity purposes, the following disclosure makes reference to slots 60, 64 and 80, and elongate tubular member 50. However, it can be appreciated that these variations may also be utilized for other slots and/or tubular members. As seen for example in Figures 5, 7 and 8, at least some, if not all of slots 60, 64 and 80 are disposed at the same or a similar angle with respect to a longitudinal axis of the elongate tubular member 50. As shown in Figures 5, 7 and 8, the slots can be disposed at an angle that is perpendicular, or substantially perpendicular, and/or can be characterized as being disposed in a plane that is normal to the longitudinal axis of the elongate tubular member 50. However, in other embodiments, the slots 60, 64 and 80 can be disposed at an angle that is not perpendicular, and/or can be characterized as being disposed in a plane that is not normal to the longitudinal axis. Additionally, a group of one or more slots 60, 64 and 80 may be disposed at different angles relative to another group of one or more slots 60, 64 and 80. The distribution and/or configuration of the slots 60, 64 and 80 can also include, to the extent applicable, any of those disclosed in U.S. Pat. Publication No. US 2004/0181174.

The slots 60, 64 and 80 may be provided to enhance flexibility while still allowing for suitable torque transmission characteristics. The slots 60, 64 and 80 may be formed such that one or more rings and/or tube segments interconnected by one or more segments and/or beams that are formed in the elongate tubular member 50, and such tube segments and beams may include portions of the elongate tubular member 50 that remain after the slots 60, 64 and 80 are formed. Such an interconnected structure may act to maintain a relatively high degree of torsional stiffness, while maintaining a desired level of lateral flexibility. In some embodiments, some adjacent slots 60, 64 and 80 can be formed such that they include portions that overlap with each other about the circumference of the elongate tubular member 50. In other embodiments, some adjacent slots 60, 64 and 80 can be disposed such that they do not necessarily overlap with each other, but are disposed in a pattern that provides lateral flexibility.

Additionally, the slots 60, 64 and 80 can be arranged along the length of, or about the circumference of the elongate tubular member 50 to achieve desired properties. For example, adjacent slots 60, 64 and 80, or groups of slots 60, 64 and 80, can be arranged in a symmetrical pattern, such as being disposed essentially equally on opposite sides about the circumference of the elongate tubular member 50, or can be rotated relative to each other about the axis of the elongate tubular member 50. Additionally, adjacent slots 60, 64 and 80, or groups of slots 60, 64 and 80, may be equally spaced along the length of the elongate tubular member 50, or can be arranged in an increasing or decreasing density pattern, or can be arranged in a non-symmetric or irregular pattern. Other characteristics, such as slot size, slot shape, and/or slot angle with respect to the longitudinal axis of the elongate tubular member 50, can also be varied along the length of the elongate tubular member 50 in order to vary the flexibility or other properties.

As suggested herein, the slots 60, 64 and 80 may be formed in groups of two, three, four, five, or more slots 60, 64 and 80, which may be located at substantially the same location along the axis of the elongate tubular member 50. Alternatively, a single slot may be disposed at some or all of these locations. Within the groups of slots 60, 64 and 80, there may be included slots that are equal in size (e.g., span the same circumferential distance around the elongate tubular member 50). In some of these as well as other embodiments, at least some slots in a group are unequal in size. Longitudinally adjacent groups of slots 60, 64 and 80 may have the same or different configurations. For example, some embodiments include slots 60, 64 and 80 that are equal in size in a first group and then unequally sized in an adjacent group. It can be appreciated that in groups that have two slots that are equal in size and are symmetrically disposed around the tube circumference, the centroid of the pair of beams (e.g., the portion of the elongate tubular member 50 remaining after slots are formed therein) is coincident with the central axis of the elongate tubular member 50. Conversely, in groups that have two slots 60, 64 and 80 that are unequal in size and whose centroids are directly opposed on the tube circumference, the centroid of the pair of beams can be offset from the central axis of the elongate tubular member 50. Some embodiments of the elongate tubular member 50 include only slot groups with centroids that are coincident with the central axis of the elongate tubular member 50, only slot groups with centroids that are offset from the central axis of the elongate tubular member 50, or slot groups with centroids that are coincident with the central axis of the elongate tubular member 50 in a first group and offset from the central axis of the elongate tubular member 50 in another group. The amount of offset may vary depending on the depth (or length) of the slots and can include other suitable distances.

The slots can be formed by methods such as micro-machining, saw-cutting (e.g., using a diamond grit embedded semiconductor dicing blade), electron discharge machining, grinding, milling, casting, molding, chemically etching or treating, or other known methods, and the like. In some such embodiments, the structure of the elongate tubular member 50 is formed by cutting and/or removing portions of the tube to form the slots. Some example embodiments of appropriate micromachining methods and other cutting methods, and structures for tubular members including slots and medical devices including tubular members are disclosed in U.S. Pat. Publication Nos. 2003/0069522 and 2004/0181174-A2; and U.S. Pat. Nos. 6,766,720 and 6,579,246. Some example embodiments of etching processes are described in U.S. Pat. No. 5,106,455.

In at least some embodiments, the slots 60, 64, 80 may be formed in the elongate tubular member 50 using a laser cutting process. The laser cutting process may include a suitable laser and/or laser cutting apparatus. For example, the laser cutting process may utilize a fiber laser. Utilizing processes like laser cutting may be desirable for a number of reasons. For example, laser cutting processes may allow the elongate tubular member 50 to be cut into a number of different cutting patterns in a precisely controlled manner. This may include variations in the slot width, ring width, beam height and/or width, etc. Furthermore, changes to the cutting pattern can be made without the need to replace the cutting instrument (e.g., blade). This may also allow smaller tubes (e.g., having a smaller outer diameter) to be used to form the elongate tubular member 50 without being limited by a minimum cutting blade size. Consequently, the elongate tubular member 50 may be fabricated for use in neurological devices or other devices where a relatively small size may be desired.

As noted, the elongate shaft 14 or portions thereof may include a polymeric covering or coating. A variety of different materials may be used. In some embodiments, a polymeric covering may include any suitable polymeric material, including biocompatible materials such as polyurethane or silicone. Other suitable polymers include but are not limited to polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b*-styrene) (for example, SIBS and/or SIBS 50A), polycarbonates, ionomers, biocompatible polymers, other suitable materials, or mixtures, combinations, copolymers thereof, polymer/metal composites, and the like.

The elongate tubular member 50 may be formed of any suitable desired material, such as a biocompatible material including biostable, bioabsorbable, biodegradable or bioerodible materials. For instance, the elongate tubular member 50 may be formed of a metallic material. Some suitable metallic materials include, but are not necessarily limited to, stainless steel, tantalum, tungsten, nickel-titanium alloys such as those possessing shape memory properties commonly referred to as nitinol, nickel-chromium alloys, nickel-chromium-iron alloys, cobalt-chromium-nickel alloys, or other suitable metals, or combinations or alloys thereof.

In some embodiments, elongate tubular member 50 may include one or more metals. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickeltungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

Those skilled in the art will recognize that the present disclosure may be manifested in a variety of forms other than the specific examples described and contemplated herein. Accordingly, departure in form and detail may be made without departing from the scope of the present disclosure as described in the appended claims.

## Claims

1. An elongate medical device comprising:
an elongate tubular body (14, 50) extending from a proximal region to a distal region and defining a lumen (22) extending there between, the elongate tubular body (14, 50) including a proximal portion (16, 54) configured for isotropic bending and a distal portion (18, 52) configured for anisotropic bending;
a first plurality of steering wire guides (94a, 94b, 94c) formed along a first side (62) of the distal region (86) of the elongate tubular body (14, 50);
a first steering wire (90) extending within the lumen (22) and having a distal end (91) secured to the distal region (86) of the elongate tubular body (14, 50), the first steering wire (90) passing through the first plurality of steering wire guides (94a, 94b, 94c) such that the first steering wire (90) is held along the first side (62) of the distal region (86);
a second plurality of steering wire guides (96a, 96b, 96c) formed along a second side (66) of the distal region (86) of the elongate tubular body (14, 50); and
a second steering wire (92) extending within the lumen (22) and having a distal end (93) secured to the distal region (86) of the elongate tubular body (14, 50), the second steering wire (92) passing through the second plurality of steering wire guides (96a, 96b, 96c) such that the second steering wire (92) is held along the second side (66) of the distal region (86);
wherein the proximal portion (16, 54) configured for isotropic bending comprises a plurality of slots (80) cut into the elongate tubular body (14, 50), the plurality of slots (80) including a first pair of opposed slots (82) at a first position on the elongate tubular body (14, 50) and a second pair of opposed slots (84) adjacent to the first pair of slots (82) and arranged at a second position axially offset and rotated from the first position; and
wherein the distal portion (18, 52) configured for anisotropic bending comprises a plurality of slots (60, 64) cut into the elongate tubular body (14, 50), the plurality of slots including a first slot (60) cut into the first side (62) of the elongate tubular body (14, 50) and a second slot (64) cut into the elongate tubular body (14, 50) from the second side (66) of the elongate tubular body (14, 50), the second slot axially offset from the first slot.

2. The elongate medical device of claim 1, wherein the first slot and the second slot each have a cutting depth that exceeds half of a diameter of the elongate tubular body (14, 50) such that the first slot overlaps the second slot, thereby forming a first bending hinge line extending along the elongate tubular body (14, 50) and a second bending hinge line extending along the elongate tubular body (14, 50).

3. The elongate medical device of any one of claims 1 or 2, wherein at least some of the first plurality of steering wire guides (94a, 94b, 94c) and/or at least some of the second plurality of steering wire guides (96a, 96b, 96c) comprise a portion of the elongate tubular body (14, 50), between adjacent slots, that is bent to form a loop extending into the lumen (22).

4. The elongate medical device of any one of claims 1 to 3, wherein the elongate tubular body (14, 50) includes a single tube forming the proximal portion (16, 54) and the distal portion (18, 52) of the elongate tubular body (14, 50).

5. The elongate medical device of claim 1, wherein the first plurality of steering wire guides (94a, 94b, 94c) is formed into the first side (62) of the distal region (86) of the elongate tubular body (14, 50) and the second plurality of steering wire guides (96a, 96b, 96c) is formed into the second side (66) of the distal region (86) of the elongate tubular body (14, 50).

6. The elongate medical device of claim 5, wherein the distal region (86) of the elongate tubular body (14, 50) comprises a plurality of slots that are cut into the elongate tubular body (14, 50).

7. The elongate medical device of claim 6, wherein at least one of the first plurality of steering wire guides formed into the first side (62) of the distal region (86) of the elongate tubular body (14, 50) are formed by bending a portion of the elongate tubular body (14, 50), between adjacent slots, into a loop extending into the lumen (22) and/or at least one of the one or more steering wire guides formed into the second side (66) of the distal region (86) of the elongate tubular body (14, 50) are formed by bending a portion of the elongate tubular body (14, 50), between adjacent slots, into a loop extending into the lumen (22).

8. The elongate medical device of claim 6, wherein the plurality of slots are formed in a pattern that provides the distal region (86) of the elongate tubular body (14, 50) with anisotropic bending.

9. The elongate medical device of any one of claims 5 to 8, wherein the proximal portion (16, 54) of the elongate tubular body (14, 50) comprises a plurality of slots that are cut into the elongate tubular body (14, 50) and are configured to provide the proximal portion (16, 54) of the elongate tubular body (14, 50) with isotropic bending.

## Patentansprüche

1. Längliche medizinische Vorrichtung, aufweisend:
einen länglichen röhrenförmigen Körper (14, 50), der sich von einem proximalen Bereich zu einem distalen Bereich erstreckt und ein Lumen (22) definiert, das sich dazwischen erstreckt, wobei der längliche röhrenförmige Körper (14, 50) einen proximalen Abschnitt (16, 54), der für isotropes Biegen konfiguriert ist, und einen distalen Abschnitt (18, 52), der für anisotropes Biegen konfiguriert ist, aufweist;
eine erste Vielzahl von Lenkdrahtführungen (94a, 94b, 94c), die entlang einer ersten Seite (62) des distalen Bereichs (86) des länglichen röhrenförmigen Körpers (14, 50) ausgebildet sind;
einen ersten Führungsdraht (90), der sich innerhalb des Lumens (22) erstreckt und ein distales Ende (91) aufweist, das an dem distalen Bereich (86) des länglichen röhrenförmigen Körpers (14, 50) befestigt ist, wobei der erste Führungsdraht (90) durch die erste Vielzahl von Lenkdrahtführungen (94a, 94b, 94c) verläuft, so dass der erste Führungsdraht (90) entlang der ersten Seite (62) des distalen Bereichs (86) gehalten wird;
eine zweite Vielzahl von Lenkdrahtführungen (96a, 96b, 96c), die entlang einer zweiten Seite (66) des distalen Bereichs (86) des länglichen röhrenförmigen Körpers (14, 50) ausgebildet sind; und
einen zweiten Führungsdraht (92), der sich innerhalb des Lumens (22) erstreckt und ein distales Ende (93) aufweist, das an dem distalen Bereich (86) des länglichen röhrenförmigen Körpers (14, 50) befestigt ist, wobei der zweite Führungsdraht (92) durch die zweite Vielzahl von Lenkdrahtführungen (96a, 96b, 96c) verläuft, so dass der zweite Führungsdraht (92) entlang der zweiten Seite (66) des distalen Bereichs (86) gehalten wird;
wobei der proximale Abschnitt (16, 54), der für isotropes Biegen konfiguriert ist, eine Vielzahl von Schlitzen (80) aufweist, die in den länglichen röhrenförmigen Körper (14, 50) geschnitten sind, wobei die Vielzahl von Schlitzen (80) ein erstes Paar von gegenüberliegenden Schlitzen (82) an einer ersten Position an dem länglichen röhrenförmigen Körper (14, 50) und ein zweites Paar von gegenüberliegenden Schlitzen (84) neben dem ersten Paar von Schlitzen (82) und an einer zweiten Position bezüglich der ersten Position axial versetzt und gedreht angeordnet aufweist; und
wobei der distale Abschnitt (18, 52), der für anisotropes Biegen konfiguriert ist, eine Vielzahl von Schlitzen (60, 64) aufweist, die in den länglichen röhrenförmigen Körper (14, 50) geschnitten sind, wobei die Vielzahl von Schlitzen einen ersten Schlitz (60), der in die erste Seite (62) des länglichen röhrenförmigen Körpers (14, 50) geschnitten ist, und einen zweiten Schlitz (64), der in den länglichen röhrenförmigen Körper (14, 50) von der zweiten Seite (66) des länglichen röhrenförmigen Körpers (14, 50) geschnitten ist, aufweist, wobei der zweite Schlitz axial von dem ersten Schlitz versetzt ist.

2. Längliche medizinische Vorrichtung nach Anspruch 1, wobei der erste Schlitz und der zweite Schlitz jeweils eine Schneidtiefe aufweisen, die die Hälfte eines Durchmessers des länglichen röhrenförmigen Körpers (14, 50) übersteigt, so dass der erste Schlitz den zweiten Schlitz überlappt, wodurch eine erste Biegescharnierlinie, die sich entlang des länglichen röhrenförmigen Körpers (14, 50) erstreckt, und eine zweite Biegescharnierlinie, die sich entlang des länglichen röhrenförmigen Körpers (14, 50) erstreckt, gebildet werden.

3. Längliche medizinische Vorrichtung nach einem der Ansprüche 1 oder 2, wobei zumindest einige der ersten Vielzahl von Lenkdrahtführungen (94a, 94b, 94c) und/oder zumindest einige der zweiten Vielzahl von Lenkdrahtführungen (96a, 96b, 96c) einen Abschnitt des länglichen röhrenförmigen Körpers (14, 50) zwischen benachbarten Schlitzen aufweisen, der gebogen ist, um eine Schleife zu bilden, die sich in das Lumen (22) erstreckt.

4. Längliche medizinische Vorrichtung nach einem der Ansprüche 1 bis 3, wobei der längliche röhrenförmige Körper (14, 50) eine einzelne Röhre aufweist, die den proximalen Abschnitt (16, 54) und den distalen Abschnitt (18, 52) des länglichen röhrenförmigen Körpers (14, 50) bildet.

5. Längliche medizinische Vorrichtung nach Anspruch 1, wobei die erste Vielzahl von Lenkdrahtführungen (94a, 94b, 94c) in die erste Seite (62) des distalen Bereichs (86) des länglichen röhrenförmigen Körpers (14, 50) gebildet ist und die zweite Vielzahl von Lenkdrahtführungen (96a, 96b, 96c) in die zweite Seite (66) des distalen Bereichs (86) des länglichen röhrenförmigen Körpers (14, 50) gebildet ist.

6. Längliche medizinische Vorrichtung nach Anspruch 5, wobei der distale Bereich (86) des länglichen röhrenförmigen Körpers (14, 50) eine Vielzahl von Schlitzen aufweist, die in den länglichen röhrenförmigen Körper (14, 50) geschnitten sind.

7. Längliche medizinische Vorrichtung nach Anspruch 6, wobei zumindest eine der ersten Vielzahl von Lenkdrahtführungen, die in die erste Seite (62) des distalen Bereichs (86) des länglichen röhrenförmigen Körpers (14, 50) gebildet sind, durch Biegen eines Abschnitts des länglichen röhrenförmigen Körpers (14, 50) zwischen benachbarten Schlitzen in eine Schleife gebildet sind, die sich in das Lumen (22) erstreckt, und/oder zumindest einer oder mehrere Lenkdrahtführungen, die in die zweite Seite (66) des distalen Bereichs (86) des länglichen röhrenförmigen Körpers (14, 50) gebildet sind, durch Biegen eines Abschnitts des länglichen röhrenförmigen Körpers (14, 50) zwischen benachbarten Schlitzen in eine Schleife gebildet sind, die sich in das Lumen (22) erstreckt.

8. Längliche medizinische Vorrichtung nach Anspruch 6, wobei die Vielzahl von Schlitzen in einem Muster gebildet ist, welches dem distalen Bereich (86) des länglichen röhrenförmig Körpers (14, 50) anisotropes Biegen verleiht.

9. Längliche medizinische Vorrichtung nach einem der Ansprüche 5 bis 8, wobei der proximale Abschnitt (16, 54) des länglichen röhrenförmigen Körpers (14, 50) eine Vielzahl von Schlitzen aufweist, die in den länglichen röhrenförmig Körper (14, 50) geschnitten sind und eingerichtet ist, dem proximalen Abschnitt (16, 54) des länglichen röhrenförmigen Körpers (14, 50) isotropes Biegen zu verleihen.

## Revendications

1. Dispositif médical allongé comprenant :
un corps tubulaire allongé (14, 50) s'étendant à partir d'une région proximale jusqu'à une région distale et définissant une lumière (22) s'étendant entre elles, le corps tubulaire allongé (14, 50) comprenant une partie proximale (16, 54) configurée pour la flexion isotrope et une partie distale (18, 52) configurée pour la flexion anisotrope ;
une première pluralité de guides de fil de guidage (94a, 94b, 94c) formée le long d'un premier côté (62) de la région distale (86) du corps tubulaire allongé (14, 50) ;
un premier fil de guidage (90) s'étendant à l'intérieur de la lumière (22) et ayant une extrémité distale (91) fixée sur la région distale (86) du corps tubulaire allongé (14, 50), le premier fil de guidage (90) passant par la première pluralité de guides de fil de guidage (94a, 94b, 94c) de sorte que le premier fil de guidage (90) est maintenu le long du premier côté (62) de la région distale (86) ;
une seconde pluralité de guides de fil de guidage (96a, 96b, 96c) formée le long d'un second côté (66) de la région distale (86) du corps tubulaire allongé (14, 50) ; et
un second fil de guidage (92) s'étendant à l'intérieur de la lumière (22) et ayant une extrémité distale (93) fixée sur la région distale (86) du corps tubulaire allongé (14, 50), le second fil de guidage (92) passant par la seconde pluralité de guides de fil de guidage (96a, 96b, 96c) de sorte que le second fil de guidage (92) est maintenu le long du second côté (66) de la région distale (86) ;
dans lequel la partie proximale (16, 54) configurée pour la flexion isotrope comprend une pluralité de fentes (80) découpées dans le corps tubulaire allongé (14, 50), la pluralité de fentes (80) comprenant une première paire de fentes opposées (82) dans une première position sur le corps tubulaire allongé (14, 50) et une seconde paire de fentes opposées (84) adjacente à la première paire de fentes (82) et agencée dans une seconde position axialement décalée et entraînée en rotation à partir de la première position ; et
dans lequel la partie distale (18, 52) configurée pour la flexion anisotrope comprend une pluralité de fentes (60, 64) découpées dans le corps tubulaire allongé (14, 50), la pluralité de fentes comprenant une première fente (60) découpée dans le premier côté (62) du corps tubulaire allongé (14, 50) et une seconde fente (64) découpée dans le corps tubulaire allongé (14, 50) à partir du second côté (66) du corps tubulaire allongé (14, 50), la seconde fente étant axialement décalée de la première fente.

2. Dispositif médical allongé selon la revendication 1, dans lequel la première fente et la seconde fente ont chacune une profondeur de coupe qui dépasse la moitié d'un diamètre du corps tubulaire allongé (14, 50) de sorte que la première fente chevauche sur la seconde fente, formant ainsi une première ligne de charnière de flexion s'étendant le long du corps tubulaire allongé (14, 50), et une seconde ligne de charnière de flexion s'étendant le long du corps tubulaire allongé (14, 50).

3. Dispositif médical allongé selon l'une quelconque des revendications 1 ou 2, dans lequel au moins certains de la première pluralité de guides de fil de guidage (94a, 94b, 94c) et/ou au moins certains de la seconde pluralité de guides de fil de guidage (96a, 96b, 96c) comprennent une partie du corps tubulaire allongé (14, 50) entre des fentes adjacentes, qui est pliée pour former une boucle s'étendant dans la lumière (22).

4. Dispositif médical allongé selon l'une quelconque des revendications 1 à 3, dans lequel le corps tubulaire allongé (14, 50) comprend un tube unique formant la partie proximale (16, 54) et la partie distale (18, 52) du corps tubulaire allongé (14, 50).

5. Dispositif médical allongé selon la revendication 1, dans lequel la première pluralité de guides de fil de guidage (94a, 94b, 94c) est formée dans le premier côté (62) de la région distale (86) du corps tubulaire allongé (14, 50) et la seconde pluralité de guides de fil de guidage (96a, 96b, 96c) est formée dans le second côté (66) de la région distale (86) du corps tubulaire allongé (14, 50).

6. Dispositif médical allongé selon la revendication 5, dans lequel la région distale (86) du corps tubulaire allongé (14, 50) comprend une pluralité de fentes qui sont découpées dans le corps tubulaire allongé (14, 50).

7. Dispositif médical allongé selon la revendication 6, dans lequel au moins l'un de la première pluralité de guides de fil de guidage formée dans le premier côté (62) de la région distale (86) du corps tubulaire allongé (14, 50) est formé en pliant une partie du corps tubulaire allongé (14, 50), entre des fentes adjacentes, en une boucle s'étendant dans la lumière (22) et/ou au moins l'un des un ou plusieurs guides de fil de guidage formés dans le second côté (66) de la région distale (86) du corps tubulaire allongé (14, 50) est formé en pliant une partie du corps tubulaire allongé (14, 50), entre des fentes adjacentes, en une boucle s'étendant dans la lumière (22).

8. Dispositif médical allongé selon la revendication 6, dans lequel la pluralité de fentes sont formées selon un motif qui dote la région distale (86) du corps tubulaire allongé (14, 50) d'une flexion anisotrope.

9. Dispositif médical allongé selon l'une quelconque des revendications 5 à 8, dans lequel la partie proximale (16, 54) du corps tubulaire allongé (14, 50) comprend une pluralité de fentes qui sont découpées dans le corps tubulaire allongé (14, 50) et sont configurées pour doter la partie proximale (16, 54) du corps tubulaire allongé (14, 50) d'une flexion isotrope.
